# EUROPEAN PATENT APPLICATION

(11) **EP 3 415 164 A1**
(43) Date of publication of application: **19.12.2018**
(21) Application number: 17382360.0
(22) Date of filing: 12.06.2017
(51) Int. Cl.: A61K 45/06, A61K 31/337, A61K 31/437, A61K 31/4418, G01N 33/574, A61P 35/00

(54) **COMPOSITION COMPRISING A SPECIFIC P38A INHIBITOR AND AN AGENT THAT INDUCES CHROMOSOME INSTABILITY AND MEDICAL USES THEREOF**

(71) Applicant: Fundació Institut de Recerca Biomèdica (IRB Barcelona), 08028 Barcelona (ES); Institució Catalana de Recerca i Estudis Avançats, 08010 Barcelona (ES)
(72) Inventor: RODRIGUEZ NEBREDA, Ángel, E-08028 Barcelona (ES); IGEA FERNÁNDEZ, Ana, E-08028 Barcelona (ES); CÁNOVAS BILBAO, Begoña, E-08028 Barcelona (ES)
(74) Representative: ABG Intellectual Property, S.L.

(57) **Abstract**

The present invention relates to compositions comprising a specific p38α inhibitor and an agent that induces chromosome instability, wherein preferably the specific p38α inhibitor has a half maximal inhibitory concentration (IC50) equal to or less than 40 nM. The invention also relates to the above compositions or to a specific p38α inhibitor for use in the treatment of breast cancer, wherein the breast cancer is characterised by a high degree of chromosome instability. Finally, the invention relates to a method for identifying a breast cancer patient who responds to a treatment with the above compositions or with a specific p38α inhibitor, wherein the breast cancer is characterised by a high degree of chromosome instability, and a method for selecting a treatment for a breast cancer patient.

## Description

### FIELD OF THE INVENTION

The present invention relates to the field of breast cancer. In particular, the present invention relates to the use of compositions comprising a specific p38α inhibitor and an agent that induces chromosome instability, and of specific p38α inhibitors for the treatment of breast cancer.

### BACKGROUND OF THE INVENTION

Breast cancer is the most frequently diagnosed cancer and the second leading cause of cancer-related death in women. It is a heterogeneous group of diseases with different histopathological characteristics and high genetic variability, and is therefore characterised by different prognostic outcomes. This diversity highlights the importance of understanding the biology of breast cancer and developing improved clinically useful classifications in terms of prognosis, prediction and therapy design.

### SUMMARY OF THE INVENTION

The inventors have surprisingly found that loss of p38α in a mouse model of breast cancer results in enhanced chromosome instability (CIN), ultimately promoting cancer cell death and tumour regression. Moreover, the inventors have found that p38α inhibitors potentiate the effects of taxanes (a type of agent that induces chromosome instability) in both genetically modified mice and in human patient-derived xenografts (PDXs) with high levels of CIN.

Thus, in a first aspect the invention relates to a composition comprising a specific p38α inhibitor and an agent that induces chromosome instability, wherein preferably the p38α inhibitor has a half maximal inhibitory concentration (IC50) equal to or less than 40 nM.

In a second aspect, the invention relates to a pharmaceutical composition comprising the composition according to the first aspect of the invention and a pharmaceutically acceptable excipient.

In a third aspect, the invention relates to a composition according to the first aspect of the invention or to a pharmaceutical composition according to the second aspect of the invention for use in the treatment of breast cancer.

In a fourth aspect, the invention relates to a method for identifying a breast cancer patient who responds to a treatment with a composition according to the first aspect of the invention or a pharmaceutical composition according to the second aspect of the invention, wherein if the tumour of said patient has a high degree of chromosome instability, the patient is identified as a responder.

In a fifth aspect, the invention relates to a method for selecting a treatment for a breast cancer patient, wherein if the tumour of said patient has a high degree of chromosome instability, the patient is selected for treatment with a composition according to the first aspect of the invention or a pharmaceutical composition according to the second aspect of the invention.

In a sixth aspect, the invention relates to a specific p38α inhibitor for use in the treatment of breast cancer, wherein the breast cancer is characterised by a high degree of chromosome instability, and/or wherein the breast cancer is a luminal breast cancer or a triple negative breast cancer.

In a seventh aspect, the invention relates to a method for identifying a breast cancer patient who responds to a treatment with a specific p38α inhibitor, wherein if the breast cancer of said patient has a high degree of chromosome instability, the patient is identified as a responder.

In an eighth aspect, the invention relates to a method for selecting a treatment for a breast cancer patient, wherein if the breast cancer of said patient has a high degree of chromosome instability, the patient is selected for treatment with a specific p38α inhibitor.

Finally, in a ninth aspect, the invention relates to a method for treating a patient suffering from breast cancer which comprises the step of administering to the patient a composition according to the first aspect of the invention, or a pharmaceutical composition according to the second aspect of the invention, or a specific p38α inhibitor, wherein the breast cancer is characterised by a high degree of chromosome instability, and/or wherein the breast cancer is a luminal breast cancer or a triple negative breast cancer.

### BRIEF DESCRIPTION OF THE FIGURES

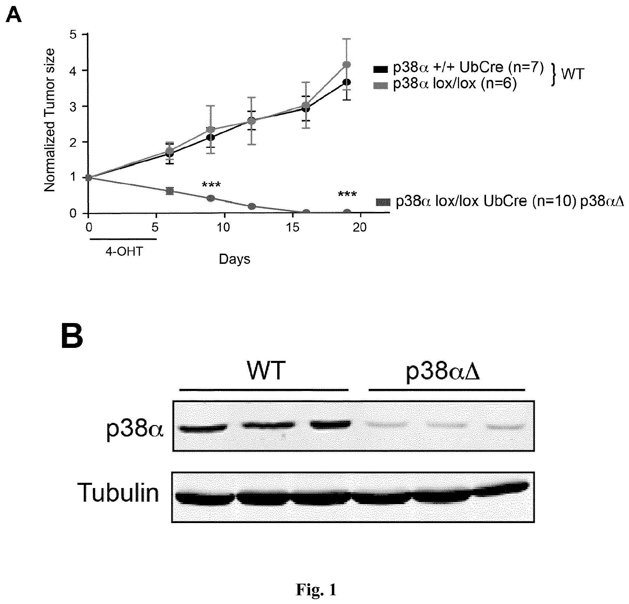

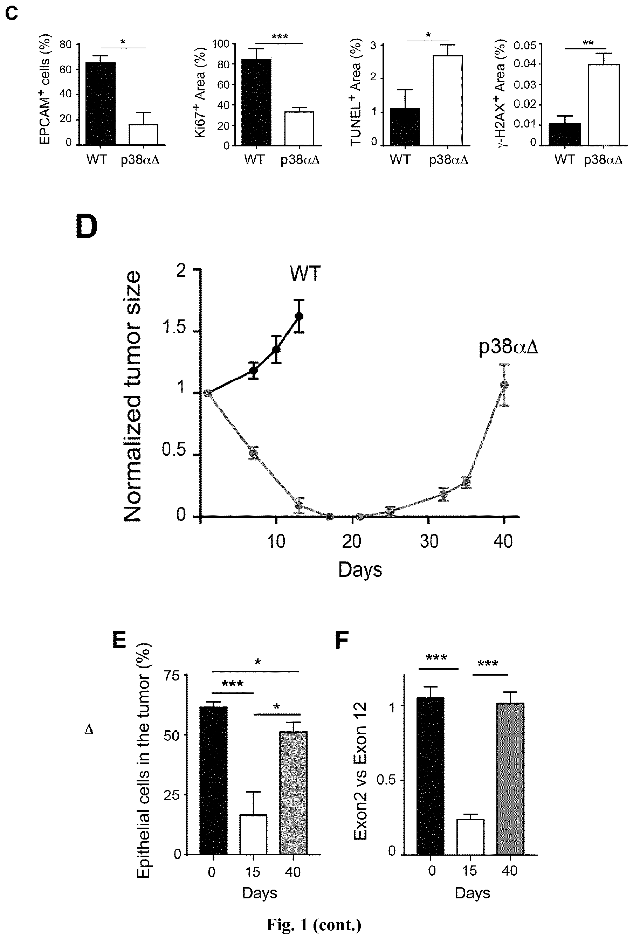

**Figure 1****: p38α is essential for PyMT-induced mammary tumour progression.** (A) Tumour growth in mice expressing MMTV-PyMT treated with 4-OHT for 5 days to induce p38α deletion in the p38α lox/lox UbCre mice. Measurements were normalized to the initial tumour size. Statistical significance was performed at days 9 and 18 using Student's test between either p38α lox/lox or p38α +/+ UbCre and p38α lox/lox UbCre. (B) Western blots with WT and p38α-deficient tumours at day 15. (C) Histograms showing the epithelial cell percentage in tumours measured by FACS (WT n=3; p38αΔ n=3), and the quantification of Ki67+ cells (WT n=3; p38αΔ n=6), TUNEL+ area (WT n=3; p38αΔ n=5) and γ-H2AX+ area (WT=5; p38αΔ n=4). At least 10 fields per tumour were analysed. Stainings were performed at day 15. (D) Tumour growth in mice expressing MMTV-PyMT treated with 4-OHT for 5 days. Measurements were normalized to the initial tumour size. Day 0, n=16; day 15, n=16; day 40, n=8. (E) Percentage of epithelial (Epcam+) cells in tumours measured by FACS using EPCAM-FITC antibody. Day 0, n=8; day 15, n=3; day 40, n=4. (F) Analysis of genomic deletion of the floxed exon2 of p38α. Day 0, n=5; day 15, n=5; day 40, n=3.

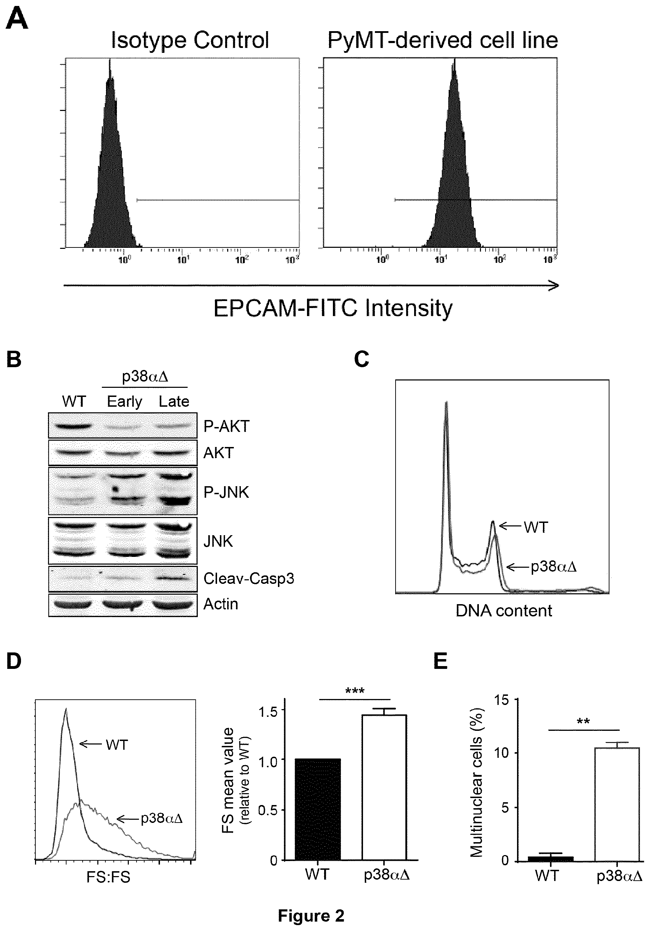

**Figure 2****: Characterization of epithelial cell lines derived from mammary tumours.** (A) EPCAM-FITC fluorescence intensity of cells derived from PyMT-induced mammary tumours and its corresponding isotype control. (B) WT and p38α-deficient cancer cells were analysed by Western blotting using the indicated antibodies. (C) Cell cycle profile analysis of WT and p38α-deficient cancer cells. (D) Representative graph of the forward-scatter (FS) of WT and p38α-deficient cancer cells. The histogram shows the values from five independent experiments. (E) The percentages of multinuclear cells in WT and p38α-deficient cancer cells were calculated using E-cadherin and DAPI staining. Histogram shows the quantification of two independent experiments.

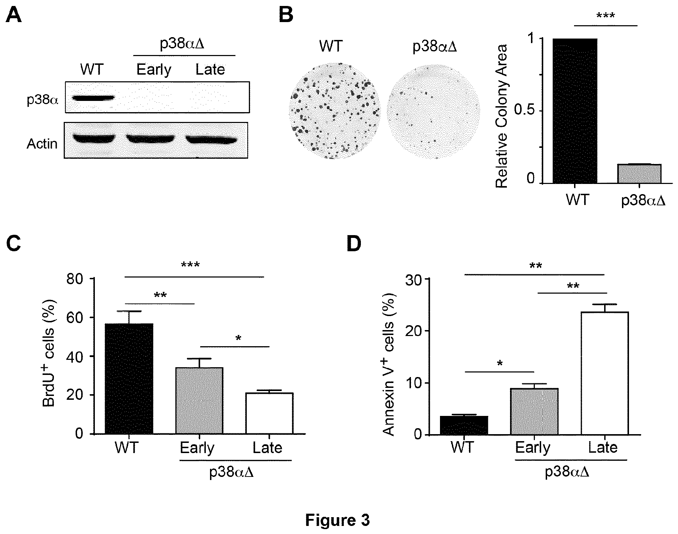

**Figure 3****: Cells derived from PyMT-induced mammary tumours reproduce the in vivo tumour behaviour.** (A) Cells were analysed by Western blotting for p38α levels. Early and Late refer to two and six days after the 4-OHT treatment, respectively. (B) Colony formation assays using WT and p38α-deficient cells. Histogram shows the quantification of two independent experiments. (C) BrdU uptake in WT and p38α-deficient cells. Histogram represents the quantification of three independent experiments. (D) Annexin staining of WT and p38α-deficient cells. Histogram represents the quantification of three independent experiments.

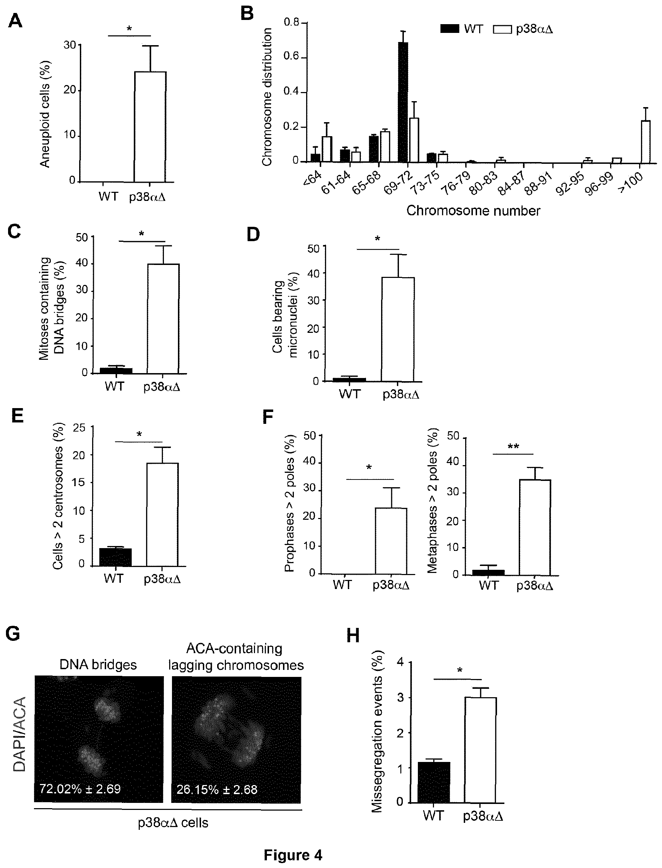

**Figure 4****: p38α deletion induces chromosome instability in cancer cells.** (A) WT and p38α-deficient cancer cells derived from PyMT-induced mammary tumours were used to prepare chromosome spreads and the number of chromosomes in at least 50 spreads was counted. Spreads containing median ±30% chromosomes were considered aneuploid. Histogram shows the quantification of three independent experiments. (B) Chromosome number distribution in WT and p38αΔ cells. Histogram shows the quantification of three independent experiments. (C) Percentage of mitoses showing DNA bridges in p38α-deficient cancer cells in three independent experiments. At least 60 mitoses per condition were analysed in each experiment. (D) Percentage of cells bearing micronuclei in two independent experiments. At least 500 cells were analysed in each experiment. (E) Percentage of cells showing > 2 centrosomes from two independent experiments. At least 500 cells were analysed in each experiment. (F) Quantification of multipolar prophases and metaphases in three independent experiments. At least 60 mitoses per condition were analysed in each experiment. (G) Representative images of DNA bridges and ACA-containing lagging chromosomes in p38α-deficient cancer cells. Values were obtained from three independent experiments were at least 20 defective mitoses were analysed. (H) Quantification of missegregation events in two independent tumours. At least 20 fields per tumour were quantified.

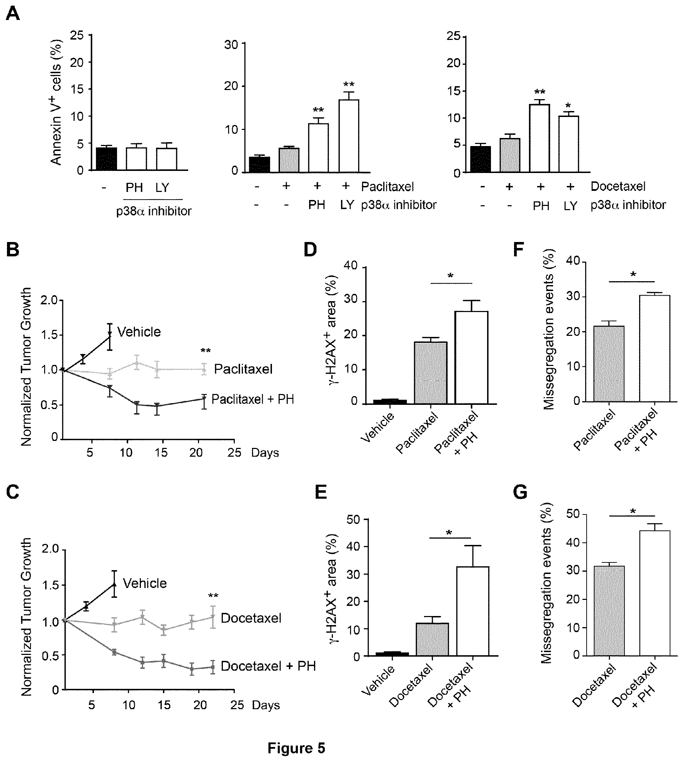

**Figure 5****: Inhibition of p38α potentiates the effect of paclitaxel and docetaxel in mammary tumours.** (A) Cancer cells derived from PyMT-induced mammary tumours were treated with the p38α inhibitors PH797804 (PH) and LY2228820 (LY), either alone (left panel) or in combination with 25 nM paclitaxel or 5 nM docetaxel (centre and right panels) for 48 h and cell death was measured by AnnexinV/PI staining. Histograms show the quantification of three independent experiments. Statistical significance was calculated between each drug alone and the drug in combination with every p38α inhibitor. (B and C) Growth curve of PyMT-induced mammary tumours in mice treated with paclitaxel or docetaxel either alone or together with PH797804. The same vehicle-treated animals are represented in both graphs. (D and E) DNA damage was measured using γ-H2AX staining. Histograms represent the γ-H2AX+ area in at least 5 independent mammary tumours, with a minimum of 15 fields quantified per tumour. (F and G) Mitotic figures were analysed at the end of the treatment using phospho-H3 staining. Histograms show the percentage of aberrant mitoses in at least two independent mammary tumours, with at least 10 fields quantified per tumour.

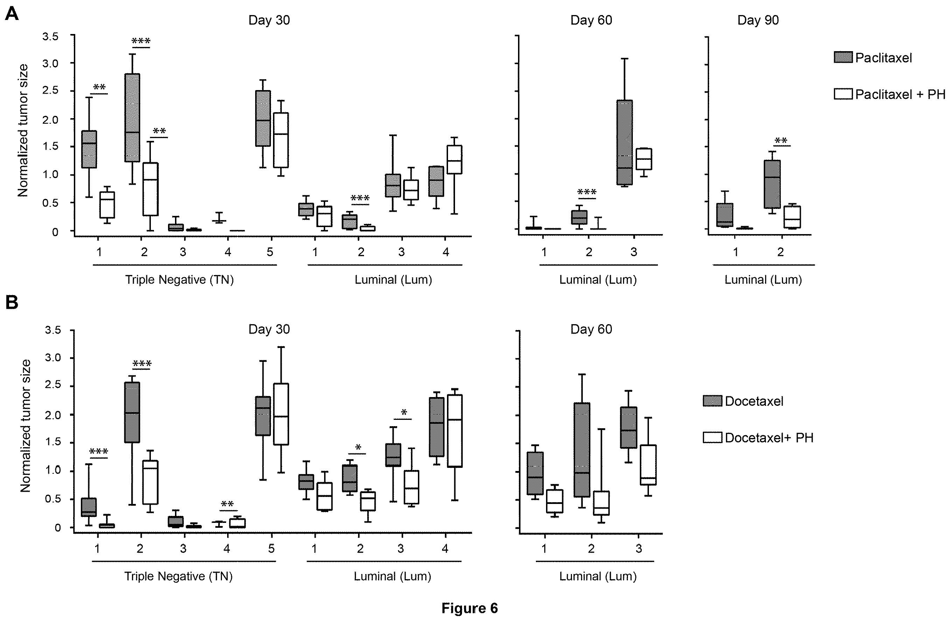

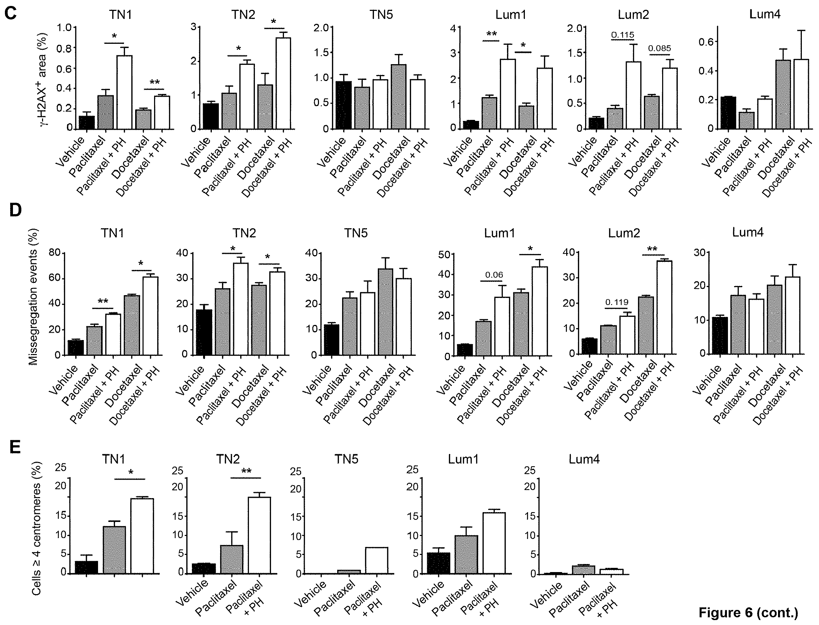

**Figure 6****: Pharmacological inhibition of p38α enhances the anti-tumour effect of paclitaxel and docetaxel in human breast tumours.** (A) NOD/Scid female mice with breast tumour patient-derived xenografts (PDXs) were treated with vehicle and paclitaxel, either alone or in combination with daily administration of the p38α inhibitor PH797804 for 21 days and tumour size was followed up. Box plots show the relative tumour size of each model and treatment at the indicated days. (B) NOD/Scid female mice with breast tumour patient-derived xenografts (PDXs) were treated with vehicle and docetaxel, either alone or in combination with daily administration of the p38α inhibitor PH797804 for 21 days and tumour size was followed up. Box plots show the relative tumour size of each model and treatment at the indicated days. (C) DNA damage was measured by γ-H2AX staining in tumour sections. γ-H2AX+ area was determined in at least 10 fields per sample. (D) Mitotic figures were analysed by phospho-H3 staining at the end of the treatment. Histograms show the percentage of aberrant mitoses in three tumours per condition, with at least 10 fields analysed per group. (E) Tumours were stained with a chromosome 17 centromeric probe, and at least 150 cells were analysed in each group. Histograms show the percentage of cells carrying 4 or more centromeres. Two independent tumours per condition were analysed except for model TN5, were data correspond to one representative tumour.

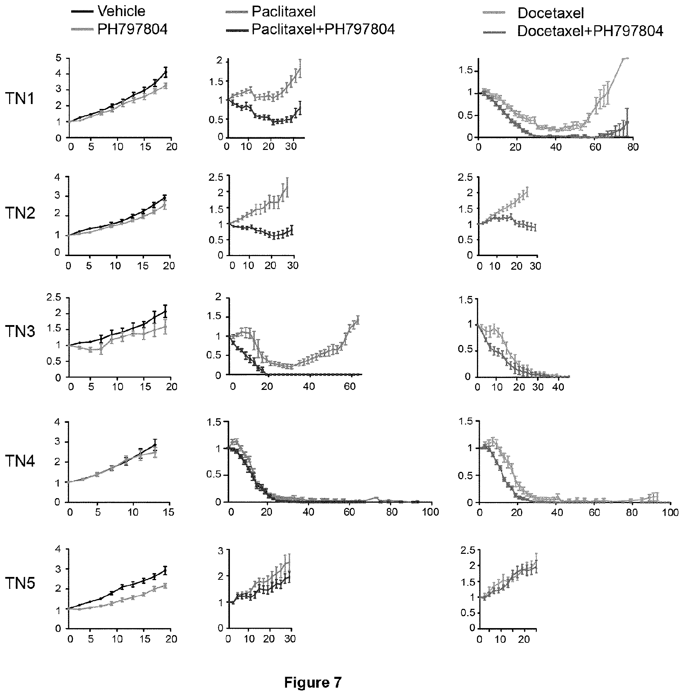

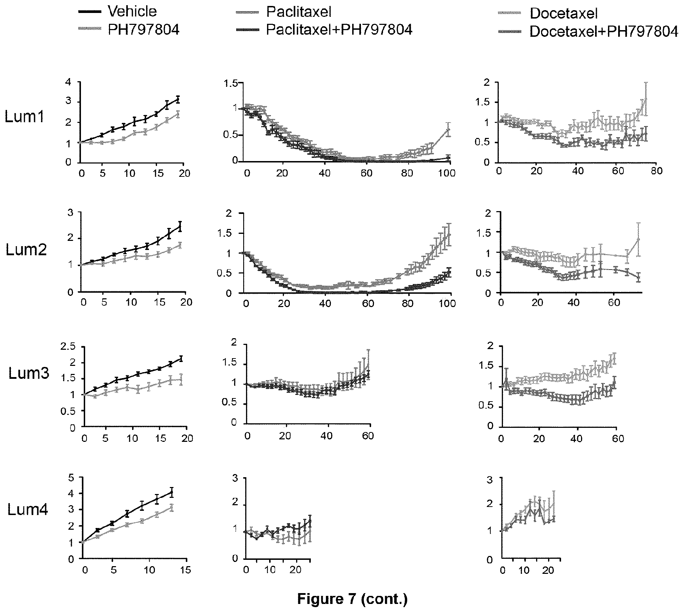

**Figure 7****: Effect of paclitaxel and docetaxel alone or in combination with PH797804 in different PDX models.** NOD/Scid female mice implanted with the indicated PDXs were treated with vehicle, the p38α inhibitor PH797804 administered daily for 21 days, and paclitaxel or docetaxel alone and in combination with PH797804, as indicated. The relative tumour growth for each treatment and PDX is shown. Tumour growth was followed up to 20 days for PH797804 alone, and up to 90 days or when one of the treatments reached twice the initial tumour size for the taxane treatments.

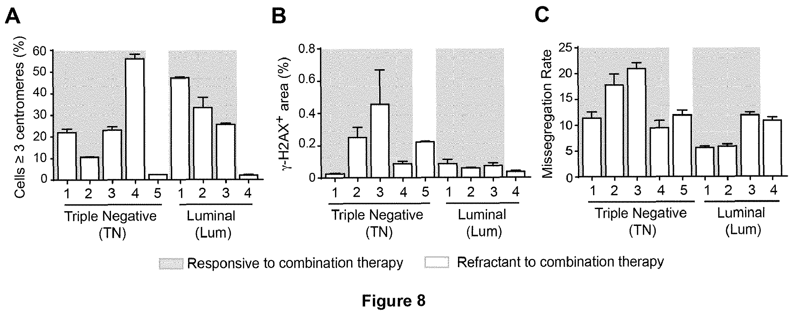

**Figure 8****: Breast tumours with high aneuploidy levels respond better to the combination of taxanes and p38α inhibition.** (A) Aneuploidy, (B) DNA damage, and (C) missegregation rates of the different PDXs were analysed in untreated tumours of 150-200 mm³. Grey backgrounds indicate breast tumours where the combination therapy effectively reduced tumour size compared to vehicle. White backgrounds indicate models where the combination therapy failed to significantly reduce tumour size.

### DETAILED DESCRIPTION OF THE INVENTION

The inventors have surprisingly found that loss of p38α in a mouse model of breast cancer results in enhanced CIN, ultimately promoting cancer cell death and tumour regression. Moreover, the inventors have found that p38α inhibitors potentiate the effects of taxanes (a type of agent that induces chromosome instability) in both genetically modified mice and in human patient-derived xenografts (PDXs) with high levels of CIN.

### Compositions comprising a Specific p38α inhibitors and an agents that induces Chromosome instability

Thus, in a first aspect the invention relates to a composition comprising a specific p38α inhibitor and an agent that induces chromosome instability. Preferably the p38α inhibitor has a half maximal inhibitory concentration (IC50) equal to or less than 40 nM.

The term "p38α", as used herein refers to the mitogen-activated protein kinase 14, also known as MAPK14, CSPB1, CSBP1, PRKM15, CSBP2, Mxi2, PRKM14, SAPK2A, p38, p38MAPK, RK. The human gene is shown in the Ensembl database under accession number ENSG00000112062.

The term "specific p38α inhibitor" as used herein, refers to a compound which is capable of inhibiting p38α with high specificity. By "high specificity" it is understood that the compound is capable of inhibiting p38α more potently than other targets, in particular other kinases, other serine/threonine kinases and/or other members of the MAPK kinase family including one or more of p38β, p38γ and/or p38δ MAPK. More preferably, specific p38α inhibitors are capable of inhibiting p38α more potently than other targets, in particular at the therapeutically effective amount used for the treatment.

A specific p38α inhibitor according to the present invention includes compounds which prevent the expression of p38α gene, compounds which lead to reduced p38α mRNA or protein levels, as well as any compounds that inhibit the activity of p38α.

The expression of a protein or nucleic acid is considered reduced when its levels decrease with respect to the reference value by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent). The reference value refers to the level of an mRNA or protein in control subject, which may be a subject who does not suffer a specific disease. In the context of the present invention, the reference value refers to the protein or mRNA level of p38α in a control subject, thus, a subject who does not suffer from breast cancer. The reference value also includes the expression level of p38α (i.e. mRNA or protein levels) in a control subject.

It is possible to determine whether a compound which leads to reduced p38α mRNA or protein levels is capable of inhibiting p38α more potently than other targets by comparing the percentage of p38α mRNA or protein level reduction.

Suitable methods for determining whether an inhibitor specific for p38α is capable of decreasing p38α mRNA levels include, without limitation, standard assays for determining mRNA expression levels such as qPCR, RT-PCR, RNA protection analysis, Northern blot, RNA dot blot, in situ hybridization, microarray technology, tag based methods such as serial analysis of gene expression (SAGE), including variants such as LongSAGE and SuperSAGE, microarrays, fluorescence in situ hybridization (FISH), including variants such as Flow-FISH, qFiSH and double fusion FISH (D-FISH), and the like.

Suitable methods for determining whether an inhibitor acts by decreasing the p38α protein levels include the quantification by means of conventional methods, for example, using antibodies with a capacity to specifically bind to the proteins encoded by p38α gene (or to fragments thereof containing antigenic determinants) and subsequent quantification of the resulting antibody-antigen complexes. There are a wide variety of well-known assays that can be used in the present invention, which use non-labelled antibodies (primary antibody) and labelled antibodies (secondary antibodies); among these techniques are included Western blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips or protein microarrays including specific antibodies or assays based on colloidal precipitation in formats such as dipsticks. Other ways of detecting and quantifying the levels of the protein of interest include techniques of affinity chromatography, binding-ligand assays, etc.

Specific p38α inhibitors acting by inhibiting the activity of the p38α protein according to the present invention include those compounds having adequate half maximal inhibitory concentration.

The term "half maximal inhibitory concentration" or (IC50) as used herein refers to the concentration of p38α inhibitor required for 50% target inhibition. Specificity of a particular inhibitor is defined as a ratio of the IC50 values of the particular inhibitor for the target of interest versus another target. Thus, it is possible to determine whether a compound is capable of inhibiting p38α more potently than other targets by comparing the IC50 values. For example, a specific p38α inhibitor will have an IC50 value for p38α lower than that for p38β. For example, the IC50 value for p38α is at least 2 times lower, at least 4 times lower, at least 6 times lower, or at least 8 times lower than the IC50 value of the same inhibitor for p38β. Moreover, a specific p38α inhibitor will have an IC50 value for p38α lower than that for other targets, including other kinases, other serine/threonine kinases and/or one or more of p38γ an/or p38δ. For example, the IC50 value for p38α is at least 10 times lower than the IC50 value of the same inhibitor for other targets. In other examples, the IC50 value for p38α is 100 times lower, or in other example is 1000 times lower. In still other example, the IC50 value for p38α is 10,000 times lower than the IC50 value of the same inhibitor for other target. In another embodiment, the specific p38α inhibitor has an IC50 for p38α which is similar to that for p38β, but lower than that for other targets, including other serine/threonine kinases or other kinases. In an embodiment, the p38α inhibitor has a half maximal inhibitory concentration (IC50) equal to or less than 40 nM, equal to or less than 35 nM, equal to or less than 30 nM, equal to or less than 25 nM, equal to or less than 20 nM, equal to or less than 15 nM, equal to or less than 10 nM, or equal to or less than 5 nM. In a particular embodiment, the IC50 value is measured by a p38 kinase enzymatic assay or by a cell-free assay. In a preferred embodiment, the IC50 value is measured by a cell-free assay (H. R. Hope et al., The Journal of Pharmacology and Experimental Therapeutics, Vol. 331, No. 3).

A specific p38α inhibitor for use in the present invention may specifically inhibit p38α activity by at least 5%, at least 10%, at least 25%, at least 50%, at least 75%, or at least 90%, and all ranges between 5% and 100%.

Suitable methods for determining whether an inhibitor acts by decreasing the p38α activity include any method which allows the determination of p38α activity levels such as by determining the phosphorylation level of any substrates which are phosphorylated by p38α such as the MAP kinase-activated protein kinase 2 (MAPKAPK2 or MK2). Assays to determine the activity of an enzyme are known by the skilled person and include, without limitation, initial rate assays, progress curve assays, transient kinetics assays and relaxation assays. Continuous assays of enzymatic activity include, without limitation, spectrophotometric, fluorometric, calorimetric, chemiluminescent, light scattering and microscale thermophoresis assays. Discontinuous assays of enzymatic activity include, without limitation, radiometric and chromatographic assays. As the skilled person understands, factors that may influence enzymatic activity comprise salt concentration, temperature, pH, and substrate concentration.

In a particular embodiment, the p38α specific inhibitor is a p38α-specific inhibitory antibody. The term "inhibitory antibody", as used herein, relates to an antibody which specifically binds to p38α and is capable of inhibiting at least partially, the biological activity of p38α. Methods for obtaining antibodies are known by the skilled in the art. The antibodies to be employed in these methods can be, for example, polyclonal sera, hybridoma supernatants or monoclonal antibodies, antibody fragments, Fv, Fab, Fab' and F(ab')2, ScFv, diabodies, triabodies, tetrabodies and humanized antibodies.

In a particular embodiment, the specific p38α inhibitor for use according to the invention is a p38α-specific RNA interference (RNAi), which is capable of knocking down the expression of p38α or of any gene necessary for p38α function. In the context of the invention, the RNAi specific for p38α may be comprised by a stable viral vector system, such as an adenovirus.

In a particular embodiment, the specific p38α inhibitor for use according to the invention is a p38α-specific short-interfering RNA (siRNA).

In a particular embodiment, the specific p38α inhibitor for use according to the invention is a p38α-specific microRNA. MicroRNAs are short single-stranded RNA molecules, typically of about 21-23 nucleotides in length capable of regulating gene expression. miRNAs may be synthetic (i.e., recombinant) or natural. Natural miRNAs are encoded by genes that are transcribed from DNA and processed from primary transcripts ("pri-miRNA") to short stem-loop structures ("pre-miRNA"), and finally to mature miRNA. Mature miRNA molecules are partially complementary to one or more mRNA molecules, and downregulate gene expression via a process similar to RNA interference, or by inhibiting translation of mRNA.

In a particular embodiment, the specific p38α inhibitor for use according to the invention is a p38α-specific antisense nucleic acid, which inhibits transcription and/or translation of p38α nucleic acid. p38α inhibiting antisense nucleic acids according to the invention include, without limitation, human p38α antisense compounds as disclosed.

In a particular embodiment, the specific p38α inhibitor for use according to the invention is a p38α-specific DNA enzyme. DNA enzymes incorporate some of the mechanistic features of both antisense and microRNA technologies. DNA enzymes are designed so that they recognize a particular target nucleic acid sequence, much like an antisense oligonucleotide, however much like a microRNA they are catalytic and specifically cleave the target nucleic acid.

In a particular embodiment, the specific p38α inhibitor is a small molecule which inhibits the activity of the p38α protein.

In an embodiment, the specific p38α inhibitor is an ATP competitor or an allosteric inhibitor (Michael S Marber et al., Drug Discov Today Dis Mech. 2010 SUMMER; 7(2): e123-e127). The term "ATP competitor" refers to inhibitors of p38α that bind competitively to the ATP pocket of the kinase. The term "allosteric inhibitor" refers to non-ATP competitive inhibitors of p38α which bind to the Asp-Phe-Gly (DFG)-motif at the base of the activation loop of p38α. Such binding exerts an allosteric effect such that the kinase adopts and maintains a conformation that is resistant to activation by dual phosphorylation.

In a preferred embodiment, the specific p38α inhibitor is an ATP competitor selected from the group consisting of PH797804, LY2228820, VX-702, VX-745, Pamapimod (R-1503, Ro4402257), BMS-582949, TAK-715, Skepinone-L, Losmapimod (GW856553X), SD-169, SD-06, SB242235, SB202190, R1487 and combinations thereof; or an allosteric inhibitor selected from the group consisting of BIRB796, Pexmetinib (ARRY-614), KR-003048 and combinations thereof (Please see Table 1).

In a preferred embodiment, the specific p38α inhibitor is selected from the group consisting of PH797804, LY2228820 and combinations thereof. In a particular embodiment, the p38α inhibitor is PH797804. In another particular embodiment, the p38α inhibitor is LY2228820.

**Table 1: Half maximal inhibitory concentration (IC50) values for different specific p38α inhibitors.**

| ***p38α inhibitor*** | ***IC50*** | ***CAS number*** | ***Reference*** |
|---|---|---|---|
| RWJ67657 | 0.5 nM | 215303-72-3 | Wadsworth S. A., J Pharmacol Exp Ther. 1999 Nov;291(2):680-7. |
| SD-169 | 3.2 nM | 1670-87-7 | Medicherla, S., et al. 2006. J. Pharmacol. Exp. Ther. 318, 99. |
| Pexmetinib (ARRY-614) | 4 nM | 945614-12-0 | Koch K., 2013 AACR Annual Meeting. |
| VX-702 | 4-20 nM | 745833-23-2 | Kuliopulos A, Thromb Haemost. 2004 Dec; 92(6):1387-93. |
| Skepinone-L | 5 nM | 1221485-83-1 | Koeberle SC, et al. Nat Chem Biol. 2011, 8(2), 141-143. |
| LY2228820 | 7 nM | 862507-23-1 | Lalaoui N. et al., Cancer Cell. 2016 Feb 8;29(2): 145-58. |
| TAK-715 | 7.1 nM | 303162-79-0 | Miwatashi S, J Med Chem. 2005 Sep 22; 48(19):5966-79. |
| SCIO 469 | 9 nM | 309913-83-5 | Nikas and Drosos. Curr Opin Investig Drugs. 2004, 5(11), 1205-1212. |
| SX 011 | 9 nM | 309913-42-6 | Lee and Dominguez, Curr Med Chem. 2005;12(25):2979-94. |
| R1487 | 10 nM | 449808-64-4 | Goldstein, J Med Chem. 2011 Apr 14; 54(7):2255-65. |
| VX-745 | 10 nM | 209410-46-8 | Zhong J. et al., Mol Cell Proteomics. 2012 Jun; 11(6): M112.017764. |
| BMS-582949 | 13 nM | 623152-17-0 | Liu C, et al. J Med Chem. 2010, 53(18):6629-6639. |
| Pamapimod (R-1503) | 14 nM | 449811-01-2 | Hill RJ, et al. J Pharmacol Exp Ther. 2008, 327(3):610-9. |
| SB242235 | 19 nM | 193746-75-7 | Badger et al., Osteoarthritis Cartilage. 2000 Nov;8(6):434-43. |
| KR-003048 | 22 nM | | A G Montalban et al., European Journal |
| | | | of Pharmacology 632 (2010) 93-102. |
| Losmapimod (GW856553X) | 25 nM | 585543-15-3 | Willette et al, J Pharmacol Exp Ther. 2009 Sep;330(3):964-70. |
| PH797804 | 26 nM | 586379-66-0 | Hope et al, J Pharmacol Exp Ther. (2009) 331:882-895. |
| JX401 | 32 nM | 349087-34-9 | Friedmann et al, Mol Pharmacol, 70(4), 1395-1405 (2006). |
| BIRB796 | 38 nM | 285983-48-4 | Kuma et al., J. Biol. Chem, Vol. 280, No. 20, pp. 19472-19479, May 2005. |
| SB202190 | 67 nM | 152121-30-7 | Karaman, et al, Nat. Biotechno. 26, 127-132. (2008). |
| SD-06 | 170 nM | 271576-80-8 | Goldstein, Curr Top Med Chem. 2005; 5(10):1017-29. |

The compositions according to the present invention comprise as second component a compound which induces CIN.

The term "chromosome instability" (CIN), as used herein, is defined by a high rate of chromosome structural and numerical abnormalities, i.e. deletion or duplication of either whole chromosomes or parts of chromosomes, leading to aneuploidy (incorrect number of chromosomes).

In an embodiment, the agent that induces chromosome instability is selected from the group consisting of an agent that disrupts microtubule dynamics, an agent that disrupts mitosis, an agent that disrupts histone deacetylase activity, an agent that disrupts the DNA and RNA metabolism, an agent that disrupts cell survival and proliferation, other agents that induce chromosome instability, and combinations thereof.

In a particular embodiment, the agent that disrupts microtubule dynamics is a microtubule stabilising agent selected from the group consisting of Ixabepilone, Peloruside A, Dactylolide, Zampanolide, Docetaxel, and Paclitaxel; or a microtubule destabilising agent selected from the group consisting of Vincristine, Nocadazole, Combretastatin A4, Maytansine, Cryptophycin 1, Vindesine, Vinorelbine, and Eribuline mesylate.

In a particular embodiment, the agent that disrupts mitosis is an aurora kinase inhibitor selected from the group consisting of VX-680, MLN8737, Reversine, and ZM-447439; an PLK1 and/or PLK3 inhibitor selected from the group consisting of GW843682, and SBE13; an Eg5 inhibitor selected from the group consisting of S-trityl-L-cysteine, and Monastrol; or a Wee1 inhibitor selected from the group consisting of PD166285, PD0407824, WEE1 inhibitor, WEE1 inhibitor II, AZD1775 (MK1775), CJM061, and ADC-730.

In a particular embodiment, the agent that disrupts histone deacetylase activity is an HDAC inhibitor selected from the group consisting of Trichostatin A, Romidepsin, Mocetinostat, Entinostat, SAHA, Panobinostat, Apicidin, Tubastatin A, Belinostat, Bufexamac, Cambinol, Sirtinol, and Niacinamide.

In a particular embodiment, the agent that disrupts the DNA and RNA metabolism is an agent that disrupts the DNA damage response selected from the group consisting of a Chk1 inhibitor selected from AZD7762, PV1162, AZD7762, SCH900776 (MK-8776), LY2603618 (IC83), LY2606368, GDC-0425, GDC-0575, PF-00477736, XL844, CEP-3891, SAR-020106, CCT-244747, Arry-575, SRA-737, Prexasertib and ONT-2409; an ATR inhibitor selected from the group consisting of AZ6738, and VE821; or a PARP inhibitor selected from the group consisting of Talazoparib, Olaparib, and Veliparib. The agent that disrupts the DNA and RNA metabolism may also be a DNA topoisomerase inhibitor selected from the group consisting of Etoposide, and Camptothecin; a DNA/RNA synthesis inhibitor selected from the group consisting of Methotrexate, Cytarabine (AraC); Cisplatin, and Bleomycin; or other agents that disrupts the DNA and RNA metabolism selected from the group consisting of Doxorubicin, Gemcitabine, and Lasonolide A.

In a particular embodiment, the agent that disrupts cell survival and proliferation is selected from the group consisting of Z-VAD; Nec1, Nec1-s; SMAC; JQ1, MK-2206; PD-0325901, SNX-2112, and 17-AAG.

In a particular embodiment, other agents that induce chromosome instability are selected from the group consisting of Indotecan (LMP400), and I-BET.

In a preferred embodiment, the agent that induces chromosome instability is an agent that disrupts microtubule dynamics. In a more preferred embodiment, the agent that induces chromosome instability is a microtubule stabilising agent. In a yet more preferred embodiment, the agent that induces chromosome instability is selected from the group consisting of paclitaxel and docetaxel. In a particular embodiment, the agent that induces chromosome instability is paclitaxel. In another particular embodiment, the agent that induces chromosome instability is docetaxel.

In preferred embodiments, the compositions according to the present invention comprise PH797804 and paclitaxel; PH797804 and docetaxel; LY2228820 and paclitaxel; LY2228820 and docetaxel; PH797804, LY2228820 and paclitaxel or PH797804, LY2228820 and docetaxel.

### Pharmaceutical composition

In a second aspect, the invention relates to a pharmaceutical composition comprising the composition according to the first aspect of the invention and a pharmaceutically acceptable excipient.

Preferred excipients to be used in the present invention include sugars, starches, celluloses, gums and proteins. In a particular embodiment, the pharmaceutical composition of the invention will be formulated as a solid pharmaceutical dosage form (for example tablets, capsules, coated tablets, granules, suppositories, sterile crystalline or amorphous solids that can be reconstituted to provide liquid forms, etc.), liquid dosage form (for example solutions, suspensions, emulsions, elixirs, lotions, ointments, etc.) or semisolid dosage form (gels, pomades, creams and the like). Examples of pharmaceutically acceptable carriers are known in prior art and include phosphate buffered saline solutions, water, emulsions, such as oil/water emulsions, different types of humectants, sterile solutions, etc. The pharmaceutical compositions of the invention may be administered by any route, including, without limitation, oral, intravenous, intramuscular, intraarterial, intramedullary, intrathecal, intraventricular, transdermal, subcutanous, intraperitoneal, intranasal, enteric, topical, sublingual or rectal route. A review of the different dosage forms of active ingredients and excipients to be used and their manufacturing processes is provided in "Tratado de Farmacia Galenica", C. Fauli and Trillo, Luzan 5, S.A. de Ediciones, 1993 and in Remington's Pharmaceutical Sciences (A.R. Gennaro, Ed.), 20th edition, Williams & Wilkins PA, USA (2000).

### Medical use of the composition

In a third aspect, the invention relates to a composition according to the first aspect of the invention or to a pharmaceutical composition according to the second aspect of the invention for use in the treatment of breast cancer.

The term "cancer" or "tumour" or "tumour disease", as used herein, refers to a broad group of diseases involving unregulated cell growth. The term is usually applied to a disease characterised by uncontrolled cell division (or by an increase of survival or apoptosis resistance) and by the ability of said cells to invade other neighbouring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. Biological processes known to be related to cancer include angiogenesis, immune cell infiltration, cell migration and metastasis. Cancers usually share some of the following characteristics: sustaining proliferative signalling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumour cells resemble, which is therefore presumed to be the origin of the tumour.

In the context of the invention, the cancer is breast cancer. The term breast cancer is commonly used as the generic name for cancers originating from breast tissue, most commonly from the inner lining of milk ducts or the lobules that supply the ducts with milk. In preferred embodiments, the compositions of the invention can be used for treating ER positive (ER+) breast cancer, ER negative (ER-) breast cancer, PR positive (PR+) breast cancer, PR negative (PR-) breast cancer, HER2 positive (HER2+) breast cancer (cancer over-expressing HER2), HER2 negative (HER2-) breast cancer (cancer expressing normal levels of HER2 or under-expressing HER2 or not expressing a detectable level of HER2), hormone receptor negative breast cancer, i.e. breast cancer with neither oestrogen nor progesterone receptors (abbreviated by ER-/PR- breast cancer); and triple negative breast cancer, i.e. breast cancer with neither oestrogen nor progesterone receptors and with normal expression/under-expression (or with the absence of detectable level of expression) of HER2 (abbreviated by ER-/PR-/HER2-breast cancer).

In another embodiment, the compositions of the invention can be used for treating a patient suffering from luminal subtype A breast cancer (ER+ and/or PR+, and HER2-), luminal subtype B breast cancer (ER+ and/or PR+, and HER2+ or HER2-), HER2+ breast cancer, Normal-like breast cancer and basal-like breast cancer (also known as triple negative breast cancer: ER-, PR-, and HER2-). In a preferred embodiment, the breast cancer is a luminal breast cancer or a triple negative breast cancer.

In additional embodiments, the breast cancer is a primary tumor. In another embodiment, the breast cancer is a metastatic tumor, including breast cancer metastasis in the lung, in the liver, in the bone or in the brain.

The compositions of the invention allow the treatment of patients having different stages of breast cancer, including patients in with stage I breast cancer, stage II breast cancer, stage III breast cancer or stage IV breast cancer and, more in particular, stage IA breast cancer, stage IB breast cancer, stage IIA breast cancer, stage IIB breast cancer, stage IIIA breast cancer, stage IIIB and stage IV breast cancer. Stages I, II, III and IV in breast cancer are defined in Greene FL, Page DL, Fleming ID, et al, editors. AJCC cancer staging manual, 6th ed. New York: Springer-Verlag, 2002. The compositions of the invention allow the treatment of patients having different stages of breast cancer, including patients with stage TXN0M0 breast cancer (wherein X is an integer from 0 to 4), stage T1N0M0 breast cancer, stage T2M0N0 breast cancer, stage T1N1M0 breast cancer, stage T2N1M0 breast cancer, stage T3N0M0 breast cancer, stage T1N2M0 breast cancer, stage T2N2M0 breast cancer, stage T3N1M0 breast cancer, stage T3N2M0 breast cancer, stage T4N0M0, stage T4N1M0 breast cancer, stage T1N3M0 breast cancer, stage T2N3M0 breast cancer, stage T3N3M0 breast cancer, stage T4N2M0 breast cancer, stage T4N3M0 breast cancer or stage TXNYM1 breast cancer, wherein X is any value from 0 to 4 and Y is any value from 0 to 3, according to the TNM classification (AJCC Cancer Staging Manual, Lippincott, 5th edition, pp. 171-180, 1997).

In a preferred embodiment, the breast cancer is characterised by a high degree of chromosome instability. The term "chromosome instability" (CIN) has been previously defined and applies equally to this aspect of the invention.

The degree of chromosomal instability can be quantified by determining the number of centromeres for one particular chromosome or several chromosomes. Paraffin-shaped tumour sections are analysed by FISH (Fluorescence in situ hybridization) using a probe that recognises the centromere of a particular chromosome. The number of centromeres per cell is counted and the result is expressed as the percentage value of aneuploid cells (i.e. cells with three or more labelled centromeres) per total cells. Other methods to quantify chromosomal instability suitable for the present invention are described elsewhere (See McGranahan et al., EMBO reports (2012) 13, 528-538).

In a particular embodiment, the breast cancer is characterised by a high degree of chromosome instability when the percentage of aneuploid cells measured by FISH is at least 3%, at least 4%, at least 5%, at least 6%, at least 7%, at least 8%, at least 9%, at least 10% per total cells in the tumour. In a preferred embodiment, the breast cancer is characterised by a high degree of chromosome instability when the percentage value of aneuploid cells measured by FISH is at least 10% per total cells in the tumour.

### Methods for identifying a breast cancer patients who respond to a treatment with the composition and for selecting a treatment for a breast cancer patient

In a fourth aspect, the invention relates to a method for identifying a breast cancer patient who responds to a treatment with a composition according to the first aspect of the invention or a pharmaceutical composition according to the second aspect of the invention, wherein if the tumour of said patient has a high degree of chromosome instability, the patient is identified as a responder.

In a fifth aspect, the invention relates to a method for selecting a treatment for a breast cancer patient, wherein if the tumour of said patient has a high degree of chromosome instability, the patient is selected for treatment with a composition according to the first aspect of the invention or a pharmaceutical composition according to the second aspect of the invention.

The term "patient" or "subject" refers to a mammal, preferably a human of any age or race. In a preferred embodiment, the subject has not been treated with chemotherapy or radiotherapy. In yet another embodiment, the patient has undergone surgical resection of the tumor.

The term "responder" shall be taken to mean a patient suffering from breast cancer wherein a treatment ameliorates or improves or prevents worsening of one or more symptoms thereof or otherwise provides therapeutic benefit wherein said change results from said treatment. In contrast, the term "non-responder" shall be taken to mean a patient suffering from breast cancer wherein a treatment does not ameliorate or improve one or more symptoms thereof or does not provide therapeutic benefit to the patient.

The term "treatment", as used herein, refers to a therapy with a composition according to the first aspect of the invention or a pharmaceutical composition according to the second aspect of the invention, which is aimed at terminating, preventing, ameliorating or reducing the susceptibility to a clinical condition as described herein. Thus, "treatment," "treating," and their equivalent terms refer to obtaining a desired pharmacologic or physiologic effect, covering the treatment of a pathological condition or disorder in a mammal, including a human. The effect may be therapeutic in terms of a partial or complete cure for a disorder and/or adverse effect attributable to the disorder. That is, "treatment" includes (1) preventing the disorder from recurring in a subject, (2) inhibiting the disorder, such as arresting its development, (3) stopping or terminating the disorder or, at least, symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, or immune deficiency.

The terms "cancer", "breast cancer", "chromosome instability", and "high degree of chromosome instability" have been described elsewhere herein and are equally applied to the present methods.

In some embodiments, patients are identified as responders to the treatment using a composition of the invention if the tumour has a percentage of aneuploid cells measured by FISH of at least 3%, at least 4%, at least 5%, at least 6%, at least 7%, at least 8%, at least 9%, at least 10% per total cells in the tumour. It will be understood that the determination of the percentage of aneuploid cells can be determined in a biopsy of the tumour, In the case of metastatic tumours, the percentage of aneuploid cells can be determined in the primary tumour, in the metastasis or in both.

In some embodiments, a treatment for a breast cancer patient based on the compositions of the invention is selected wherein the tumour in the patient has a percentage of aneuploid cells measured by FISH of at least 3%, at least 4%, at least 5%, at least 6%, at least 7%, at least 8%, at least 9%, at least 10% per total cells in the tumour. It will be understood that the determination of the percentage of aneuploid cells can be determined in a biopsy of the tumour, In the case of metastatic tumours, the percentage of aneuploid cells can be determined in the primary tumour, in the metastasis or in both.

### Medical use of a specific p38α inhibitor

In a sixth aspect, the invention relates to a specific p38α inhibitor for use in the treatment of breast cancer, wherein the breast cancer is characterised by a high degree of chromosome instability, and/or wherein the breast cancer is a luminal breast cancer or a triple negative breast cancer.

Suitable specific p38α inhibitors for use according to this aspect of the invention have been defined in the context of the first aspect of the invention.

In an embodiment, the specific p38α inhibitor has a half maximal inhibitory concentration (IC50) equal to or less than 40 nM, equal to or less than 35 nM, equal to or less than 30 nM, equal to or less than 25 nM, equal to or less than 20 nM, equal to or less than 15 nM, equal to or less than 10 nM, or equal to or less than 5 nM. In a preferred embodiment, the specific p38α inhibitor has a half maximal inhibitory concentration (IC50) equal to or less than 40 nM.

In a preferred embodiment, the specific p38α inhibitor is an ATP competitor selected from the group consisting of PH797804, LY2228820, VX-702, VX-745, Pamapimod (R-1503, Ro4402257), BMS-582949, TAK-715, Skepinone-L, Losmapimod (GW856553X), SD-169, SD-06, SB242235, SB202190, R1487 and combinations thereof; or an allosteric inhibitor selected from the group consisting of BIRB796, Pexmetinib (ARRY-614), KR-003048 and combinations thereof (Please see Table 1 above). In a preferred embodiment, the p38α inhibitor is selected from the group consisting of PH797804, LY2228820 and combinations thereof In a particular embodiment, the specific p38α inhibitor is PH797804. In another particular embodiment, the p38α inhibitor is LY2228820.

In a particular embodiment, the breast cancer is characterised by a high degree of chromosome instability when the percentage value of aneuploid cells measured by FISH is at least 3%, at least 4%, at least 5%, at least 6%, at least 7%, at least 8%, at least 9%, at least 10% per total cells in the tumour. In a preferred embodiment, the breast cancer is characterised by a high degree of chromosome instability when the percentage value of aneuploid cells measured by FISH is at least 10% per total cells in the tumour.

The terms "p38α", "specific p38α inhibitor", "half maximal inhibitory concentration", "cancer", "breast cancer", "chromosome instability", and "high degree of chromosome instability" have been described elsewhere herein and are equally applied to the present aspect of the invention.

### Method for identifying a breast cancer patients who respond to a treatment with a specifc p38α inhibitor and method for selecting a treatment for a breast cancer patient

In a seventh aspect, the invention relates to a method for identifying a breast cancer patient who responds to a treatment with a specific p38α inhibitor, wherein if the breast cancer of said patient has a high degree of chromosome instability, the patient is identified as a responder.

In an eighth aspect, the invention relates to a method for selecting a treatment for a breast cancer patient, wherein if the breast cancer of said patient has a high degree of chromosome instability, the patient is selected for treatment with a specific p38α inhibitor.

In an embodiment, the specific p38α inhibitor has a half maximal inhibitory concentration (IC50) equal to or less than 40 nM, equal to or less than 35 nM, equal to or less than 30 nM, equal to or less than 25 nM, equal to or less than 20 nM, equal to or less than 15 nM, equal to or less than 10 nM, or equal to or less than 5 nM. In a preferred embodiment, the specific p38α inhibitor has a half maximal inhibitory concentration (IC50) equal to or less than 40 nM.

In a preferred embodiment, the specific p38α inhibitor is an ATP competitor selected from the group consisting of PH797804, LY2228820, VX-702, VX-745, Pamapimod (R-1503, Ro4402257), BMS-582949, TAK-715, Skepinone-L, Losmapimod (GW856553X), SD-169, SD-06, SB242235, SB202190, R1487 and combinations thereof; or an allosteric inhibitor selected from the group consisting of BIRB796, Pexmetinib (ARRY-614), KR-003048 and combinations thereof (Please see Table 1 above). In a preferred embodiment, the p38α inhibitor is selected from the group consisting of PH797804, LY2228820 and combinations thereof. In a particular embodiment, the specific p38α inhibitor is PH797804. In another particular embodiment, the p38α inhibitor is LY2228820.

In a particular embodiment, the breast cancer is characterised by a high degree of chromosome instability when the percentage value of aneuploid cells measured by FISH is at least 3%, at least 4%, at least 5%, at least 6%, at least 7%, at least 8%, at least 9%, at least 10% per total cells in the tumour. In a preferred embodiment, the breast cancer is characterised by a high degree of chromosome instability when the percentage value of aneuploid cells measured by FISH is at least 10% per total cells in the tumour.

The terms "p38α", "specific p38α inhibitor", "half maximal inhibitory concentration", "cancer", "breast cancer", "chromosome instability", and "high degree of chromosome instability" have been described elsewhere herein and are equally applied to the present aspects of the invention.

In some embodiments, patients are identified as responders to the treatment using a specific p38α inhibitor if the tumour has a percentage of aneuploid cells measured by FISH is at least 3%, at least 4%, at least 5%, at least 6%, at least 7%, at least 8%, at least 9%, at least 10% per total cells in the tumour. It will be understood that the determination of the percentage of aneuploid cells can be determined in a biopsy of the tumour, In the case of metastatic tumours, the percentage of aneuploid cells can be determined in the primary tumour, in the metastasis or in both,

In some embodiments, a treatment for a breast cancer patient based on a specific p38α inhibitor is selected wherein the tumour in the patient has a percentage of aneuploid cells measured by FISH is at least 3%, at least 4%, at least 5%, at least 6%, at least 7%, at least 8%, at least 9%, at least 10% per total cells in the tumour. It will be understood that the determination of the percentage of aneuploid cells can be determined in a biopsy of the tumour, In the case of metastatic tumours, the percentage of aneuploid cells can be determined in the primary tumour, in the metastasis or in both,

### Method of treating a patient suffering from breast cancer

Finally, in a ninth aspect, the invention relates to a method for treating a patient suffering from breast cancer which comprises the step of administering to the patient a composition according to the first aspect of the invention, or a pharmaceutical composition according to the second aspect of the invention, or a specific p38α inhibitor, wherein the breast cancer is characterised by a high degree of chromosome instability, and/or wherein the breast cancer is a luminal breast cancer or a triple negative breast cancer.

In an embodiment, the specific p38α inhibitor has a half maximal inhibitory concentration (IC50) equal to or less than 40 nM, equal to or less than 35 nM, equal to or less than 30 nM, equal to or less than 25 nM, equal to or less than 20 nM, equal to or less than 15 nM, equal to or less than 10 nM, or equal to or less than 5 nM. In a preferred embodiment, the p38α inhibitor has a half maximal inhibitory concentration (IC50) equal to or less than 40 nM.

In a preferred embodiment, the specific p38α inhibitor is an ATP competitor selected from the group consisting of PH797804, LY2228820, VX-702, VX-745, Pamapimod (R-1503, Ro4402257), BMS-582949, TAK-715, Skepinone-L, Losmapimod (GW856553X), SD-169, SD-06, SB242235, SB202190, R1487 and combinations thereof; or an allosteric inhibitor selected from the group consisting of BIRB796, Pexmetinib (ARRY-614), KR-003048 and combinations thereof (Please see Table 1 above). In a preferred embodiment, the p38α inhibitor is selected from the group consisting of PH797804, LY2228820 and combinations thereof In a particular embodiment, the p38α inhibitor is PH797804. In another particular embodiment, the p38α inhibitor is LY2228820.

In a particular embodiment, the breast cancer is characterised by a high degree of chromosome instability when the percentage value of aneuploid cells measured by FISH is at least 3%, at least 4%, at least 5%, at least 6%, at least 7%, at least 8%, at least 9%, at least 10% per total cells in the tumour. In a preferred embodiment, the breast cancer is characterised by a high degree of chromosome instability when the percentage value of aneuploid cells measured by FISH is at least 10% per total cells in the tumour.

The terms "p38α", "specific p38α inhibitor", "half maximal inhibitory concentration", "cancer", "breast cancer", "chromosome instability", and "high degree of chromosome instability" have been described elsewhere herein and are equally applied to the present aspect of the invention.

The invention is described below by means of the following examples which are merely illustrative and by no means limiting for the scope of the invention.

### EXAMPLES

### Material and Methods

**Mouse experiments.** PyMT p38αlox/lox UbCre female mice were generated by crossing p38αlox/lox mice (Ventura et al., 2007) with MMTV-PyMT, provided by William Muller (McGill University, Canada) and UbiquitinC-Cre-ERT2 mice (Ruzankina et al., 2007), being all mostly in FVB background. Mouse genotyping was performed by PCR using genomic tail DNA. Primers and conditions are available upon request. Mice were housed according to national and European Union regulations, and protocols were approved by the animal care and use committee of the Barcelona Science Park (PCB-CEEA). Breast tumours were monitored twice a week with a caliper using the formula V= (π x length) x wide2 and experiments were started when tumours reached 150-200 mm3, usually around 2.5-3 months old. For p38α deletion, 4OHT (Sigma, H6278) was dissolved in 10% ethanol/90% corn oil (Sigma, C8267) and mice were injected intraperitoneally with 1 mg each day for five consecutive days. For chemotherapeutic treatments, mice were injected once a week with 20 mg/kg of docetaxel (Accord, 691719.0) or 20 mg/kg paclitaxel (Accord, 676253.0), for three consecutive weeks. The p38α inhibitor PH797804 (Selleckchem) was dissolved in 0.05% methylcellulose in PBS and administered daily at 15 mg/kg by oral gavage.

**Patient derived xenografts (PDXs).** Models TN1 (IDB-01), TN3 (IDB-09/1835), TN5 (IDB-02) and LUM1 (IDB-04) were generated by Dr. Eva Gonzalez-Suarez with tumours from the Hospital Universitari de Bellvitge (Barcelona), which were obtained at time of surgery or thoracocentesis with informed written patient consent according to Spanish regulations. Models TN2 (VHIO #94), TN5 (VHIO #60), LUM2 (VHIO #191), LUM3 (VHIO #131) and LUM 4 (VHIO #244) were generated by Dr. Violeta Serra with tumours from the Hospital Universitari Vall d'Hebron (Barcelona) that belong to a collection with the registration number C0003435 (Josep Tabernero Caturla). Tumours were harvested when they reached a size of 1500 mm3 and tumour tissue was cut into 2- to 3-mm3 pieces in Foetal Bovine Serum (FBS). Under anaesthesia with isoflurane, one tumour piece was implanted orthotopically in the mammary fad pad by a small incision into 5-6 weeks old female NOD/Scid mice (Harlan Laboratories). For the experiments, xenografts were allowed to grow until they reached a size of 150-200 mm3 and then mice were randomized for treatments. Tumour size was measured every two days by a digital caliper using the following formula: Tumour volume = (length × width2)/2. The p38α inhibitor PH797804 (Selleckchem) was dissolved in vehicle (0.05% methylcellulose in PBS) and administered at 15 mg/kg daily by oral gavage for 20 days. For chemotherapeutic treatments, mice with TN1 and TN4 models were injected once a week with 15 mg/kg of docetaxel (Accord, 691719.0), and animals implanted with the rest of the models with 20 mg/kg of docetaxel for four consecutive weeks. All models were injected once a week with 20 mg/kg paclitaxel (Accord, 676253.0), for three consecutive weeks. Samples were collected at the indicated points and at the end of the experiment. Protocols were approved by the animal care and use committee of the Barcelona Science Park (PCB-CEEA).

**Histology and immunohistochemistry.** Tumours dissected from mice were fixed overnight in 10%-buffered formalin (Sigma, HT501128) and embedded in paraffin. Sections (5 µm-thick) were stained with hematoxylin and eosin (H&E). Specific antibodies were used for the following stainings: Ki67 (Novacastra, NCL), phospho-H3 (Millipore, 06-570), γ-H2AX (Millipore, 05-636). HRP-conjugated secondary antibodies (ImmunoLogic) were used and the signal was visualised using DAB (3,3-diaminobenzedine). Apoptosis was detected by TUNEL using the "In situ Cell Death Detection Kit, Fluorescein" (Roche, 11684795910), following the manufacturer's instructions.

**FISH.** Sections of 5 mm were deparaffined, autoclaved in citrate solution and digested with proteinase K. After formalin fixation and dehydration, samples were denatured for 5 min at 83°C and hybridized with a chromosome 17 centromeric probe (Empire Genomics CHR17-10-GO) at 37°C overnight. Then, samples were washed with prewarmed buffer, washed again at RT and air-dried. Finally, samples were mounted using mounting media containing DAPI (Life Technologies, P36935).

**Isolation of epithelial cells for p38α deletion analysis.** At the indicated times, mice were sacrificed and breast tumours were dissected, chopped using razors and digested at 37°C rocking for 1 h in DMEM medium containing Collagenase A (1 mg/ml) (Roche, 10103586001) and Hyaluronidase (1.5 units/ml) (Sigma, H3506). After digestion, the cell suspension was filtered through a 70 µm cell strainer and centrifuged 5 min at 1500 rpm. Cell pellets were resuspended in Hanks Balance media containing 1 mM HEPES and 1% FBS and digested first with 1 ml trypsin (Sigma, T3924) for 3-5 min and then with 0.4 mg DNAse (Sigma, D4513) for 2-3 min. Cells were washed again with Hank Balance media and cell density was quantified. 100.000 cells were stained using APC-CD45 (BD, 559864) for leukocytes, PE-Pdgfrα (eBioscience, 12-1401-81) for fibroblasts and EPCAM-FITC (Santa Cruz, sc- 53532) for epithelial cells. Cells were stained for 30 min on ice, washed in PBS and resuspended in PBS containing DAPI. EPCAM(+) cells were sorted in NID buffer (50 mM KCl, 10 mM Tris pH 8.3, 2 mM MgCl2, 0.1 mg/ml gelatin, 0.45% NP40, 0.45% Tween20, 1 mg/ml Proteinase K) to isolate genomic DNA. Cells were digested overnight at 56°C and after inactivation of the proteinase K at 95°C for 10 min, 2 µl of the lysate were used for the qPCR reaction. Deletion levels were analysed using specific primers for the exon 2 (floxed) and exon 12 (control) of the p38α gene. Deletion rate was calculated as the relative amount of exon 2 versus exon 12 as described (Gupta et al., 2014).

**Western blotting.** Cell lysates were obtained by trypsinization and cell centrifugation at 1400 rpm for 5 min. Cell pellets were suspended in lysis buffer (50 mM Tris HCl pH 7.5, 150 mM NaCl, 2 mM EDTA, 1%NP40, 2 mM PMSF, 2 µM mycrocystin, 2 mM sodium orthovanadate, 1 mM DTT and 1x EDTA-free complete protease inhibitor cocktail (Roche, 11873580001), mixed by vortexing and incubated 10 min on ice. Lysates were subjected to sonication, incubated on ice for 15 min and centrifuged at 13200 rpm for 15 min at 4°C. Mouse tissues were dissected, immersed in lysis buffer, and homogenized using Precellys homogenization and lysis instrument (Bertin Technologies). Lysates were incubated 15 min on ice and centrifuged 15 min at 13200 rpm at 4°C. Cell and tissue lysates were quantified using the RC DCTM Protein Assay kit II (Bio- Rad, 5000122) with BSA as standard. Total protein lysates (20-40 µg) were separated on 10-15% polyacrylamide gels and transferred onto nitrocellulose membranes (Whatman, 10401396). Membranes were blocked in 5% non-fat milk in PBS for 1h at RT and then incubated at 4°C overnight with the primary antibody. After washing with PBS, membranes were incubated with Alexa Fluor 680-conjugated secondary antibodies (Invitrogen 1:5000) for 1h at RT and visualised using Odyssey Infrared Imaging System (Li-Cor, Biosciences). The following antibodies were obtained from Cell Signalling Technology: p38α (9218), phospho-AKT S473 (9271), and Caspase 3 (9665). The antibodies for AKT (1619), and JNK (571) were obtained from Santa Cruz, phospho-JNK T183/Y185 from BD biosciences (612145). Antibodies for tubulin (Sigma, T9026) and actin (Abcam, 49846) were used as loading controls.

**Cell lines derived from PyMT-induced breast tumours.** Tumours were digested as described above for epithelial cell isolation. After digestion, cell suspension was filtered through a 70 µm cell strainer and centrifuged 5 min at 1500 rpm. The cell pellets was resuspended in 10 ml DMEM and centrifuged at 1500 rpm for 30 sec. Supernatant was removed and the cell pellet was resuspended and spun again four more times. The final cell pellet enriched in epithelial cells was plated. Cells were passaged until spontaneously immortalised and then experiments were performed. Epithelial status of the immortalised cells was confirmed by FACS using EPCAM-FITC (Santa Cruz, sc-53532). Epithelial tumour cell lines were derived from different PyMT p38α^{lox/lox UbCre} mice.

**Cell culture and treatments.** Immortalised cells derived from PyMT-induced breast tumours were maintained in Dulbecco's Modified Eagles's Media (DMEM) (Sigma, D5796) supplemented with 10% FBS, 1% L-glutamine (Labclinics x0550-100) and 1% penicillin/streptomycin (Labclinics, L0022-100). Cells were passaged every other day and routinely tested for mycoplasma using MycoAlert Detection Kit (Lonza). For Cre-ERT2 induction, 100 nM 4-OHT (Sigma, H7904) diluted in 100% ethanol was added to the culture media. 48 h later, cells were washed and split normally. Unless otherwise indicated, experiments were always performed 48 h after the removal of 4-OHT. For drug treatments, cells were treated for 48 h with docetaxel (Accord, 691719.0) or paclitaxel (Sigma, T7191) with or without different compounds that inhibit p38α: PH 797804 2 µM (Selleckchem) or LY2228820 100 nM (Selleckchem).

**Cell Cycle Analysis.** Cells were harvested and fixed in 70% cold ethanol. Cells were then incubated in PBS containing 20µg/ml propidium iodide (Sigma, P4864), 0.1 mg/ml RNaseA (Roche, 10109142001) and 0.1% Tween 20 for 30 min at 37°C in the dark. Cells were subjected to FACS analysis and cell cycle distribution was analysed with the FlowJo software.

**BrdU uptake.** Cells were pulsed with 10 mM BrdU (Roche, 10280879001) for 2 h and fixed in 70% cold ethanol. DNA was denaturized with 0.1 M HCl and then heated at 95°C for 5 min. S-phase cells were stained using the BD Pharmigen FITC BrdU Flow Kit (BD Biosciences, 559619). Cells were then resuspended in PBS containing 20 mg/ml propidium iodide and 0.1 mg/ml RNaseA and subjected to FACS analysis. The percentage of BrdU(+) cells was analysed with FlowJo software.

**Annexin V staining.** Both attached and floating cells were collected, washed in PBS and stained with FITC Annexin V Apoptosis Detection kit I (BD Pharmigen, 556547), following manufacturer's instructions. The percentage of Annexin V(+) cells was analysed with FlowJo software.

**Clonogenic assays.** WT and p38α KO cells were trypsinised and 1200 cells were plated in 60 mm dishes. Cells were allowed to grow until visible colonies were formed (about one week). Then cells were fixed in 4% paraformaldehyde (Aname, 15710) and stained with crystal Violet (Sigma, HT90132). The colony area was measured using Fiji. For drug treatments, cells were treated with the indicated drugs and inhibitors for 48 h. Then, cells were trypsinised and 1200 cells were plated in 60 mm dishes. Cells were allowed to grow for about 10 days and processed as described previously.

**Immunofluorescence.** Cells grown on coverslips were treated as indicated, washed in PBS and fixed in 4% paraformaldehyde for 20 min at RT. Then, cells were permeabilised in 0.1% Triton X-100 in PBS and blocked in 1% BSA in PBS for 1h at RT. In case of RPA, a pre-extraction step using detergent was performed in order to remove the soluble fraction. Primary antibodies were diluted in blocking buffer and incubated overnight at 4°C. Cover slips were washed with PBS and subsequently incubated with 1:400 Alexa-conjugated secondary antibodies (Invitrogen). Finally, cells were prepared using mounting media containing DAPI (Life Technologies, P36935), and were visualised using a Nikon E1000 epifluorescence microscope, a Leica TCS SPE confocal microscope or a SCANR confocal microscope. The following antibodies were used: E-cadherin (BD, 610181), α-tubulin (Sigma, T9026), γ-tubulin (Sigma, T6557) and ACA (Antibodies Incorporated, 15-235).

**Metaphase spread preparation.** Cells were treated with 100 ng/ml colcemide (Sigma, 10295892001) for 2 h and trypsinised. Pelleted cells were hypotonically swollen in 75 mM KCl for 20 min at 37°C and fixed in fixative solution (ice cold 75% methanol/25% acetic acid). After three washes in fixative solution, cells were spread on glass slides, steam treated for 5 sec, heat dried and stained with mounting media containing DAPI. Spreads were imaged using a Nikon E1000 epifluorescence microscope. Chromosome number and chromosome aberrations were analysed using Fiji software.

**Statistical Methods.** Data, unless indicated otherwise, are presented as mean±SEM. Statistical significance was determined by Student's test using Graph Pad Prism software. p-values < 0.05 (*), < 0.1 (**) and < 0.01 (***) were considered statistically different. Statistical analysis in PDX models was performed using R programming language. For all time points volumes were divided by the size of the corresponding lesion at time 0 of treatment. A boxcox transformation was applied to the normalised volumes using lambda equal to 0.23. For each time point a linear model was fitted with experimental batch and mouse as nested random effects using the "lmer" function from the lme4 R package. Coefficients and p-values for the comparisons of interest were computed through the "glht" function from the multcomp (Hothorn et al., 2008) R package using the "Westfall" multiplicity adjustment method. Confidence intervals for the coefficients were calculated with the "confint" function. Confidence intervals for the median volume for each group were computed using the "sim" function from the arm R package with 1000 simulations.

### EXAMPLE 1: Deletion of p38α induces mammary tumours regression in mice

To investigate the influence of p38α in breast tumour progression *in vivo,* the inventors combined the polyoma middle T (PyMT) mouse model with conditional alleles of p38α. PyMT tumours reflect both histological and biological features of the human disease and model some human breast cancer characteristics associated with poor-outcome, such as the loss of estrogen and progesterone receptors (Lin et al., 2003).

Mice expressing the PyMT transgene were crossed with mice carrying p38αlox/lox and the inducible UbiquitinC-CreERT2 (UbCre) alleles. Once mammary tumours were developed, p38αlox/lox UbCre mice were treated with 4-hydroxytamoxifen (4-OHT) to induce Cre activation and p38α deletion (p38αΔ). the inventors observed a progressive tumour regression following p38α deletion and found that by day 15, p38αΔ mice showed no palpable tumour masses, while their PyMT-expressing WT littermates, either p38α+/+ UbCre or p38αlox/lox treated with 4-OHT, showed continuous tumour growth (Figure 1A). The efficient downregulation of p38α in the mammary tumours from p38αΔ mice was confirmed by Western blotting (Figure 1B).

Histological analysis of the mammary fat pads revealed that although p38αΔ mice had no palpable tumours, their mammary tissue was not completely normal. These mice showed mainly hyperplastic tissue and small carcinoma areas, while WT littermates displayed histology more consistent with advanced tumour stages. The mammary tumours induced by PyMT expression are driven by the hyperproliferation of the epithelial cells of the mammary ducts. In agreement with the reduced tumour size, the mammary tissue of p38αΔ mice showed a 75% reduction in the epithelial cell compartment compared with WT mice (Figure 1C). Moreover, the inventors found decreased cell proliferation (Ki67+) and increased cell death (TUNEL+) in the mammary tissue of p38αΔ mice (Figure 1C), which is consistent with the tumour regression observed upon p38α deletion. the inventors also observed a four-fold increase in the levels of phosphorylated histone variant H2AX, referred to as γ-H2AX, which is considered a marker of DNA double-strand breaks (DSBs) (Fig. 1C), indicating that p38α downregulation results in higher levels of DNA damage.

### EXAMPLE 2: p38α is required for breast tumours cell homeostasis and Cancer progression

When p38αlox/lox UbCre mice were followed for longer times after 4-OHT treatment, the inventors noticed that tumours eventually started to re-grow (Figure 1D) and, as expected, tumour growth correlated with an increased epithelial cell compartment (Figure 1E). The inventors sorted the epithelial cells from tumours at different time points and examined the deletion of p38α at the genomic level. At day 15, when no tumours were palpable, around 25% of the remaining epithelial cells contained the floxed p38α exon 2, indicating the existence of a residual cell population that expressed p38α. However, by day 40, when tumours had re-emerged, most of the epithelial cell population contained the exon 2 (Figure 1F). These results indicate that p38α deletion was not induced in 100% of the epithelial tumour cells, and cells that retained p38α expression had a competitive advantage to grow and repopulate the tumours.

### EXAMPLE 3: Tumour-derived Epithelial cell culture reproduce key features of breast tumours

To investigate the molecular mechanisms underlying mammary tumour regression, the inventors established epithelial cell lines from PyMT-induced tumours in p38αlox/lox UbCre mice. The derived cell lines grew well, showed a typical epithelial morphology and were 100% positive for the epithelial marker EPCAM (Figure 2A). Therefore, the cell lines provided a relevant system to analyse the molecular basis for the enhanced tumour cell death and reduced tumour size observed upon p38α deletion *in vivo.*

Treatment of cultured tumour cells with 4-OHT led to efficient p38α deletion (Figure 3A) and strongly reduced the ability of the cells to form colonies, confirming that p38α was essential for the growth and/or viability of the tumour cells (Figure 3B). This correlated with decreased levels of DNA replication, which was evident at early time points (Figure 3C), and increased cell death, more evident at later time points (Figure 3D). Biochemical analysis supported these results, as the inventors observed the downregulation of the proliferation-associated AKT pathway and the upregulation of JNK signalling and cleaved-caspase 3, both indicative of increased cell death (Figure 2B). Analysis of cell cycle profiles confirmed a decrease in the S-phase population of p38αΔ cells compared to WT cells (Figure 2C), correlating with the reduced BrdU uptake (Figure 3C). Moreover, p38α deletion resulted in larger cells (Figure 2D), which more frequently exhibited multinucleation (Figure 2E) than WT cells, suggesting potential defects in cell division. These results indicated that p38α was required for breast tumour cells to maintain efficient cell cycle progression and prevent cell death.

### EXAMPLE 4: Deletion of p38α leads to increase CIN in tumours Epithelial cells

The larger cells and the higher multinucleation rate observed upon p38α downregulation suggested potential defects during chromosome segregation, which can give rise to aneuploidy. To address this, the inventors analysed chromosome numbers in metaphase spreads and found increased levels of aneuploidy in p38αΔ cells compared with WT cells (Figure 4A and 4B). Immunofluorescence-based quantifications showed that both DNA bridges and micronuclei were strongly increased in p38αΔ cells (Figure 4C and 4D), as well as other CIN features such as increased number of centrosomes (Figure 4E) and multipolar prophases and metaphases (Figure 4F). Taken together, p38α deletion in the PyMT-derived breast cancer cells resulted in a variety of mitotic aberrations, abnormal cytokinesis, increased aneuploidy and a CIN phenotype.

In order to analyse the origin of these mitotic defects, the inventors stained p38αΔ cells with an anti-centromere antibody (ACA). Structural chromosome aberrations that arise due to pre-mitotic defects are characterized by DNA bridges and chromosome fragments without centromeres, while lagging chromosomes with centromeres often suggest mitotic dysfunction such as merotelic kinetochore attachments (Burrell et al., 2013). Immunofluorescence in p38αΔ cells identified DNA bridges in most of the aberrant mitoses, but only 26% of the lagging chromosomes stained for ACA (Figure 4G). Therefore, although the inventors cannot rule out some contribution of mitotic dysfunction, their results indicated that the observed segregation errors were primarily a consequence of pre-mitotic defects, such as the DNA damage derived from impaired DNA-damage response or increased replication stress. Finally, the inventors confirmed the occurrence of CIN upon p38α downregulation *in vivo* by analysing mitotic figures in mammary tumour sections. The inventors observed an increased percentage of missegregation events in tumour cells upon p38α downregulation (Figure 4H), supporting a role for p38α in the suppression of CIN not only *ex-vivo* in cell cultures, but also *in vivo* in mouse tumours.

### EXAMPLE 5: Inhibition of p38α potentiates the anti-tumour effect of CIN-inducing chemotherapeutic drugs

Some chemotherapeutic agents used in breast cancer therapy, such as the taxanes paclitaxel and docetaxel, cause missegregation in proliferating cells and promote CIN. The results suggested that targeting p38α could increase tumour cell sensitivity to these compounds and enhance their anti-tumour effect. To test this hypothesis the inventors used two chemical inhibitors of p38α at the lowest dose that inhibits activity. In the short treatment used in these experiments, these inhibitors showed very little cytotoxic effect on the cells. However, while treatment with the inhibitors alone had a minor effect, the combination of p38α inhibition with either paclitaxel or docetaxel consistently led to significantly increased levels of apoptotic cell death (Figure 5A).

Next, the inventors validated their results using female mice with PyMT-induced mammary tumours, which were treated with paclitaxel or docetaxel and PH797804, a p38α inhibitor that has been used in clinical trials (Goldstein et al., 2010). When administered alone, the chemotherapeutic drugs only exerted a cytostatic effect on tumour growth. However, in combination with PH797804, the inventors observed a substantial reduction of tumour size (Figure 5B and 5C), which correlated with increased levels of DNA damage (Figure 5D and 5E) and a higher number of missegregation events (Figure 5F and 5G). These results are consistent with the idea that p38α contributes to the DNA damage response and facilitates the survival of breast cancer cells with high levels of CIN.

To further support the potential clinical application of their results, the inventors used nine PDXs generated from patients with triple negative or luminal tumours, which were treated with either paclitaxel or docetaxel and PH797804. The inventors found that tumour behaviour was distinct, depending on both the drug and the PDX model, highlighting the clinical diversity of human breast cancer. However, in most cases, even if p38α inhibition had a mild effect on tumour growth, co-administration of PH797804 enhanced, accelerated or prolonged the anti-tumour response observed with the taxane alone, except for TN5 and Lum4, where no additional benefit was observed (Figure 6A, 6B and 7). It is important to mention that although PH797804 did not boost the taxane effect in two out of nine PDXs, the inventors never observed increased tumour growth or any other detrimental outcome induced by p38α inhibition. The inventors then selected four tumours that responded well to the combined treatment (TN1, TN2, Lum1 and Lum2) and two in which the p38α inhibitor did not affect the taxane response (TN5 and Lum4), and analysed them immunohistochemically at an early time-point (around day 15). Consistent with their previous results in the PyMT-induced mammary tumours, PDXs in which PH797804 enhanced the anti-tumour response to taxanes showed both increased γ-H2AX staining (Figure 6C) and increased percentage of missegregation events (Figure 6D) in response to the combination therapy compared to the single treatments. Of note, no significant differences in tumour size were detected at this early stage in Lum1 and Lum2 suggesting that the enhanced DNA damage and missegregation events reflect an early response mechanism that translates into noticeable effects at later time-points (Figure 6A and 7). FISH analysis further confirmed these results, as the inventors observed that PH797804 usually enhanced the aneuploidy induced by paclitaxel in tumours (Figure 6E). Taken together, it seems that even in tumours where chemotherapy is particularly effective, interfering with p38α signalling further damages cancer cells, avoiding or delaying tumour relapse. This revealed a correlation between the aneuploidy induction in the tumour cells and the reduction in tumour growth induced by the combined treatment. Of note, no differences in DNA damage or segregation were detected in models TN5 and Lum4, where inhibiting p38α had no evident benefit.

To try to more clearly understand why p38α inhibition did not potentiate the taxane effects in models TN5 and Lum4, the inventors performed FISH in untreated tumours of every model and observed that those with a medium to high aneuploidy level responded better to combined taxane and p38α inhibition, while tumours with more stable genomes were refractory to the added effects of p38α inhibition (Figure 8A). These results suggest that the combination of p38α inhibition with taxane-based chemotherapy would be especially beneficial in those tumours that have higher CIN levels. On the other hand, the inventors found no correlation between basal levels of DNA damage or missegregation rates in the tumour and the ability of PH797804 to potentiate taxane effects (Figure 8B and 8C). Altogether, their results indicate that p38α activity contributes to the DDR and restrains CIN in human breast cancer cells derived from luminal and triple negative tumours.

## Claims

1. A composition comprising a specific p38α inhibitor and an agent that induces chromosome instability.

2. The composition according to claim 1 wherein the specific p38α inhibitor has a half maximal inhibitory concentration (IC50) equal to or less than 40 nM.

3. The composition according to claims 1 or 2 wherein the specific p38α inhibitor is selected from the group consisting of an ATP competitor, an allosteric inhibitor and combinations thereof

4. The composition according to any one of claims 1 to 3 wherein the specific p38α inhibitor is selected from the group consisting of PH797804, LY2228820 and combinations thereof.

5. The composition according to any one of claims 1 to 4 wherein the agent that induces chromosome instability is selected from the group consisting of an agent that disrupts microtubule dynamics, an agent that disrupts mitosis, an agent that disrupts histone deacetylase activity, an agent that disrupts the DNA and RNA metabolism, an agent that disrupts cell survival and proliferation, other agents that induce chromosome instability, and combinations thereof

6. The composition according to any one of claims 1 to 5 wherein the agent that induces chromosome instability is an agent that disrupts microtubule dynamics.

7. The composition according to any one of claims 1 to 6 wherein the agent that induces chromosome instability is selected from the group consisting of Paclitaxel and Docetaxel.

8. A pharmaceutical composition comprising the composition according to any one of claims 1 to 7 and a pharmaceutically acceptable excipient.

9. A composition according to any one of claims 1 to 7 or a pharmaceutical composition according to claim 8 for use in the treatment of breast cancer.

10. The composition or pharmaceutical composition for use according to claim 9, wherein the breast cancer is **characterised by** a high degree of chromosome instability.

11. The composition or pharmaceutical composition for use according to any one of claims 9 or 10, wherein the breast cancer is a luminal breast cancer or a triple negative breast cancer.

12. A method for identifying a breast cancer patient who responds to a treatment with a composition according to any one of claims 1 to 7, with a pharmaceutical composition according to claim 8 or with a specific p38α inhibitor, wherein if the tumour of said patient has a high degree of chromosome instability, the patient is identified as a responder.

13. A method for selecting a treatment for a breast cancer patient, wherein if the tumour of said patient has a high degree of chromosome instability, the patient is selected for treatment with a composition according to any one of claims 1 to 7, with a pharmaceutical composition according to claim 8 or with a specific p38α inhibitor.

14. A specific p38α inhibitor for use in the treatment of breast cancer, wherein the breast cancer is **characterised by** a high degree of chromosome instability, and/or wherein the breast cancer is a luminal breast cancer or a triple negative breast cancer.

15. The p38α inhibitor for use according to claim 14 wherein the specific p38α inhibitor is selected from the group consisting of consisting of an ATP competitor, an allosteric inhibitor and combinations thereof.

16. The specific p38α inhibitor for use according to claim 15 wherein the p38α inhibitor is selected from the group consisting of PH797804, LY2228820 and combinations thereof.
